# EUROPEAN PATENT APPLICATION

(11) **EP 0 762 124 A2**
(43) Date of publication of application: **12.03.1997**
(21) Application number: 96113163.8
(22) Date of filing: 16.08.1996
(51) Int. Cl.: G01N 33/574, G01N 33/68, G01N 30/00

(54) **Cartridge column for detecting active tgf-beta1, and method for detecting cancer using this column**

(30) Priority: 12.09.1995 JP 234354/95
(71) Applicant: MITSUBISHI MATERIALS CORPORATION, Chiyoda-ku, Tokyo (JP)
(72) Inventor: Watanabe, Etsuko, c/o Mitsubishi Mater. Corp., Omiya-shi, Saitama-ken (JP)
(74) Representative: Türk, Gille, Hrabal, Leifert

(57) **Abstract**

Disclosed is a cartridge column (1) for simple and effective detection of active TGF-β1 in a liquid sample such as a body fluid sample. Also disclosed is a method for detecting cancer using the cartridge column. Using the cartridge column (1), active TGF-β1 which is the essential substance to cause the self-secretory propagation of cancer cells can be detected. The cartridge column (1) is equipped with an inlet (2) and an outlet (3) for a liquid sample. A connecting means (7) is provided at the inlet, via which the inlet can be connected to the tip of a syringe. An ultrafilter (4) is provided at the inlet and an adsorbent dispersion-preventing filter (5) at the outlet. An adsorbent (6) for active TGF-β1 which comprises, as the active ingredient, an OH-carbonated hydroxyapatite with OH groups being partly or wholly substituted with CO₃ groups is present between the ultrafilter (4) and the filter (5). To detect cancer, a body fluid sample is introduced into the cartridge column through the inlet and diffused therein to make the sample brought into contact with the adsorbent for active TGF-β1 that exists in the cartridge column, whereupon the active TGF-β1 existing in the sample is selectively adsorbed by the adsorbent, then the thus-adsorbed, active TGF-β1 is eluted with a buffer, and the active TGF-β1 in the resulting eluate is detected.

## Description

The present invention relates to a cartridge column for detecting active TGF-β₁, comprising an adsorbent specific to active TGF-β₁ which is known to be an essential substance that may cause the self-secretory propagation of cancer cells, and also to a method for detecting cancer using the cartridge column.

Various studies have heretofore been made to develop medicines for curing cancer. In the medical treatment of cancer, the problem of how to deal with the immunodepression to be caused by chemotherapy with medicines and even by cancer itself shall be taken into consideration. In addition, the means of diagnosing cancer, including early detection of cancer prior to the treatment thereof, and also early detection of metastasis and redevelopment, if any, of cancer during and after the treatment thereof, are important in the treatment of cancer. To detect cancer, in general, there has heretofore been employed a method of directly examining the morphology of the cells existing in the tissue of the affected part as taken out from a patient. According to this method, it is often difficult to determine whether the affected part is benign or malignant prior to the operation on the part. Apart from this, there has been proposed another method of specific immunoserodiagnosis of cancer by measuring a cancer-specific glycoprotein as a detection marker (for example, see JP-C-62-100661)

The tumor marker which has heretofore been employed in the detection of cancer includes many physiologically-active substances that exist in living bodies. There has been found a correlation in some degree between the content of the substances and disorders. In fact, however, many of the substances are often found to exist even in normal cells or are often difficult to differentiate from those derived from normal cells in quality. In particular, cancer markers which are effective both quantitatively and qualitatively are not yet found at present.

On the other hand, the blood of healthy persons contains inactive TGF-β₁, which is activated when brought into contact with cancer cells, then self-secreted and promotes the propagation of cancer cells. It is known that cancer cells produce active TGF-β₁ and that inactive TGF-β1 is activated by enzymes and others, thereby increasing the concentration of active TGF-β₁ in blood and depressing the immunity to cancer. However, it is not easy to measure the concentration of minor TGF-β₁ existing in body fluids such as blood, and it is more difficult to selectively measure the concentration of active TGF-β₁ therein.

At present, to detect cancer by measuring the concentration of TGF-β₁ in blood, ELISA (enzyme-linked immunosorbent assay) as disclosed in JP-A- 5-336990 has been put into practical use.

However, ELISA is complicated and troublesome and takes much time of at least 3 days, and it is expensive. In addition, since TGF-β₁ in a sample is all activated in the pre-treatment step in ELISA, clinical examinations through ELISA are problematic in that the data obtained are often unreliable.

One object of the present invention is to provide a cartridge column which is simply and effectively usable in the detection of active TGF-β₁.

Another object of the present invention is to provide a reliable method of detecting cancer, where active TGF-β₁ which is the essential substance to cause the self-secretory propagation of cancer cells is detected by the use of the cartridge column.

According to the present invention, there is provided a cartridge column for detecting active TGF-β₁ as equipped with an inlet and an outlet for a liquid sample, which is characterized in that it has an ultrafilter as disposed at the inlet and has an adsorbent dispersion-preventing filter as disposed at the outlet and that an adsorbent for active TGF-β₁ which comprises, as the active ingredient, an OH-carbonated, hydroxyapatite with OH groups being partly or wholly substituted with CO₃ groups is present between the ultrafilter and the filter.

As one preferred embodiment of the invention, the cartridge column for detecting active TGF-β₁ is provided with a means of connecting it to the tip of a syringe, at the inlet for a liquid sample.

According to the present invention, there is also provided a method for detecting cancer using the cartridge column, in which a body fluid sample is introduced into the cartridge column through the inlet and diffused therein to make the sample brought into contact with the adsorbent for active TGF-β₁ that exists in the cartridge column, whereupon the active TGF-β₁ existing in the sample is selectively adsorbed by the adsorbent, then the thus-adsorbed, active TGF-β₁ is eluted with a buffer, and the active TGF-β₁ in the resulting eluate is detected.

The invention is more in detail explained in the following description of the attached drawings, in which
Fig. 1 is a cross-sectional view showing one embodiment of the cartridge column of the present invention, and
Fig. 2(a) and Fig. 2(b) are graphs indicating the results of the assay of the eluate resulting from the elution of three serum samples as collected from a cancer patient and those of the assay of the eluate resulting from the elution of three serum samples as collected from a healthy person, respectively, in Example 1, in which an ultraviolet detector (at 280 nm) was used for detecting the adsorbed active TGF-β₁ in each eluate.

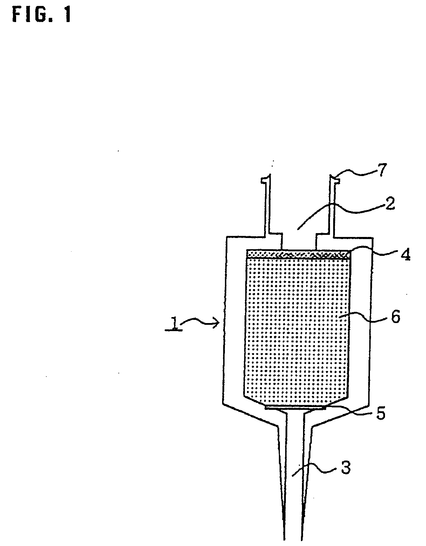

The cartridge column 1 for detecting active TGF-β1 shown in Fig. 1 is provided with an inlet 2 for a liquid sample at its top and an outlet 3 for the sample at its bottom, and this may be made of any ordinary material such as polypropylene. The inlet 2 may optionally be provided with a connecting means 7, via which the inlet 2 is connected to the tip of a syringe, and a liquid sample can be introduced into the cartridge column 1 through the syringe. The connecting means 7 may be integrated with the cartridge column 1 or, alternatively, may be removably fitted thereinto. The connecting means 7 may have any known form of, for example, a lure lock or the like.

In the inside of the cartridge column 1, an ultrafilter 4 is provided below the inlet 2 and a filter 5 above the outlet 3. Through the ultrafilter 4, it is possible to remove the impurities, which are larger than the molecules of TGF-β₁, from the sample being introduced into the cartridge column 1. The ultrafilter 4 is not specifically defined provided that TGF-β₁ having a molecular weight of about 25,000 can pass therethrough. In general, a membrane having a sieving molecular size of 30 kDa or the like can be used as the ultrafilter 4. By the provision of the filter 5, it is possible to prevent the dispersion of the adsorbent to be described later. Any ordinary filter usable in constructing the column of this type can be used as the filter 5.

An adsorbent layer 6 is provided in the space between the ultrafilter 4 and the filter 5 in the cartridge column 1. Namely, the adsorbent layer 6 is interposed between the ultrafilter 4 and the filter 5. The capacity of the space, that is, the capacity of the cartridge column 1, is not specifically defined but, in general, may be in the range from 1.5 to 4 ml. In Fig. 1, the adsorbent layer 6 is seen completely filled with an adsorbent without leaving any gap but actually it is not always required to be so and it is sufficient for the adsorbent layer to be interposed between the ultrafilter 4 and the filter 5 within the cartridge column 1.

The adsorbent to be in the adsorbent layer 6 shall be an adsorbent for active TGF-β1, which comprises, as the active ingredient, an OH-carbonated, hydroxyapatite (Ca₁₀(PO₄)₆(CO₃)ₓ(OH)₂₋₂ₓ) with OH groups being partly or wholly substituted with CO₃ groups (hereinafter referred to as adsorbent A). Preferably, the OH-carbonated hydroxyapatite has a CO₃ group content of 2.0 % by weight or more relative to its total weight.

The OH-carbonated hydroxyapatite selectively adsorbs only active TGF-β₁. It is believed that the essential mechanism of the selective adsorbent A is owing to the difference in the fine, three-dimensional structure between the extremely porous surface of the OH-carbonated hydroxyapatite therein and the local surfaces of the molecules of active TGF-β₁, and that the specific adsorption of the adsorbent A can be realized due to the difference in the local molecular structure therebetween.

It is desirable that the OH-carbonated hydroxyapatite is granular or powdery so that it can easily be filled into the cartridge column of the present invention. It is also desirable that the granular or powdery OH-carbonated hydroxyapatite comprises particles having a mean particle size of from 3 to 20 µm. This is because when an adsorbent comprising such particles having a mean particle size of 3 µm or larger is present in the cartridge column, the pressure loss in the cartridge column, which shall contain a filter and through which a liquid sample is passed, can be prevented from being too large. On the other hand, when an adsorbent comprises such particles having a mean particle size of 20 µm or smaller, the surface area of the particles per the unit volume is sufficiently broad so that the cartridge column containing the adsorbent can exhibit excellent separability. However, even if the mean particle size of the particles constituting the adsorbent to be in the cartridge column of the invention is outside the scope as defined above, it is possible to attain acceptable results.

To prepare the OH-carbonated hydroxyapatite for use in the present invention, any known methods can be employed. For example, hydroxyapatite is heated in carbon dioxide gas under normal pressure at about 1000°C or higher. To granulate or powder it, employable are any known methods of, for example, granulation through spray-drying, granulation through grinding, rotary granulation, etc.

To detect cancer according to the method of the present invention, a body fluid sample is first introduced into the cartridge column of the invention via the inlet and diffused towards the outlet, while the sample is brought into contact with the adsorbent A for active TGF-β₁ that exists in the cartridge column and thus the active TGF-β₁ existing in the sample is selectively adsorbed by the adsorbent A. Concretely, a body fluid sample is collected in a syringe, the tip of the syringe is fitted into the inlet of the cartridge column, and the piston of the syringe is pressed to make the sample injected into the cartridge column under pressure. In that manner, the sample is passed through the ultrafilter and then the adsorbent A, whereby the active TGF-β₁ existing in the sample is concentrated and adsorbed by the adsorbent A. One example of the body fluid sample may be human or animal blood. It is desirable to use, as the body fluid sample, human or animal serum and/or plasma.

Next, the active TGF-β₁ as adsorbed by the adsorbent A in the cartridge column is eluted with a buffer. The buffer may be a phosphate buffer such as KH₃PO₄ buffer (pH 7.4). The buffer may have any concentration with no specific limitation, provided that the active TGF-β₁ as adsorbed by the adsorbent A can be eluted with the buffer. Preferably, however, the buffer has a concentration of from 200 to 350 mM. The amount of the buffer and the means of injecting it into the cartridge column of the invention are not specifically defined. For example, when 350 mM KH₃PO₄ buffer is used, about 0.5 ml of the buffer is injected via a syringe and diffused in the cartridge column to attain the intended elution. Prior to the elution, KH₃PO₄ buffer having a lower ionic strength or having a concentration of about 80 mM is injected into the cartridge column, with which other proteins that have been adsorbed by the adsorbent A more weakly than the active TGF-β₁ can be removed.

Next, the eluate produced by the elution is collected, and the active TGF-β₁ contained in the thus-collected eluate is detected and its concentration is measured. The measurement of the active TGF-β₁ content can be conducted, for example, through ultraviolet absorptiometry or colorimetry using a color reagent for protein.

Since the cartridge column of the present invention contains therein an adsorbent that comprises, as the active ingredient, an OH-carbonated hydroxyapatite and is provided with an ultrafilter at the inlet, a liquid sample, such as a body fluid sample collected from a human or animal, can be directly introduced thereinto, without being pre-treated, in order to make the active TGF-β₁ that exists in the sample selectively adsorbed by the adsorbent. According to the method for detecting cancer of the present invention that uses the cartridge column, it is possible to directly introduce a body fluid sample, as collected from a human or animal, into the cartridge column, without pre-treating the sample, and the active TGF-β₁ that exists in the sample can be selectively adsorbed by the adsorbent contained in the cartridge column. The thus-adsorbed active TGF-β₁ can be eluted with a buffer. Therefore, even if the active TGF-β₁ content of the body fluid sample is minor, it can be effectively determined by simple operation. Being different from the conventional ELISA method, the method of the present invention does not require any pre-treatment of the sample to be assayed. According to the method of the invention, therefore, it is possible to detect cancer easily and distinctly within a short period of time.

The present invention will be described in more detail by means of the following example and comparative example, which, however, are not intended to restrict the scope of the present invention.

### Example 1:

A particulate, OH-carbonated, hydroxyapatite having a mean particle size of 12.3 µm, in which 3.4 % by weight, relative to the total weight of the apatite, of OH groups have been substituted with CO₃ groups, was used herein as an adsorbent for active TGF-β₁. 0.5 g of the adsorbent was suspended in 5 ml of pure water, and the resulting suspension was put into the cartridge column 1 (30 mm x 8 mm φ) (shown in Fig. 1) through spontaneous precipitation. The cartridge column was provided with an ultrafilter having a sieving molecular size of 30 kDa.

Three serum samples of 200 µl each as collected from a cancer patient and three serum samples of 200 µl each as collected from a healthy person were separately injected into the cartridge columns as prepared in the above, using syringes. Next, 80 mM KH₃PO₄ buffer (pH 7.4) was introduced into the cartridge columns, whereby impurities and proteins other than active TGF-β₁ were removed from the samples. Next, 0.5 ml of 350 mM KH₃PO₄ buffer (pH 7.4) was introduced into each cartridge column, whereby the substance as adsorbed by the adsorbent was eluted out. The substance thus eluted out into each eluate was detected, using an ultraviolet detector (at 280 nm). The eluates were collected and subjected to the next assay.

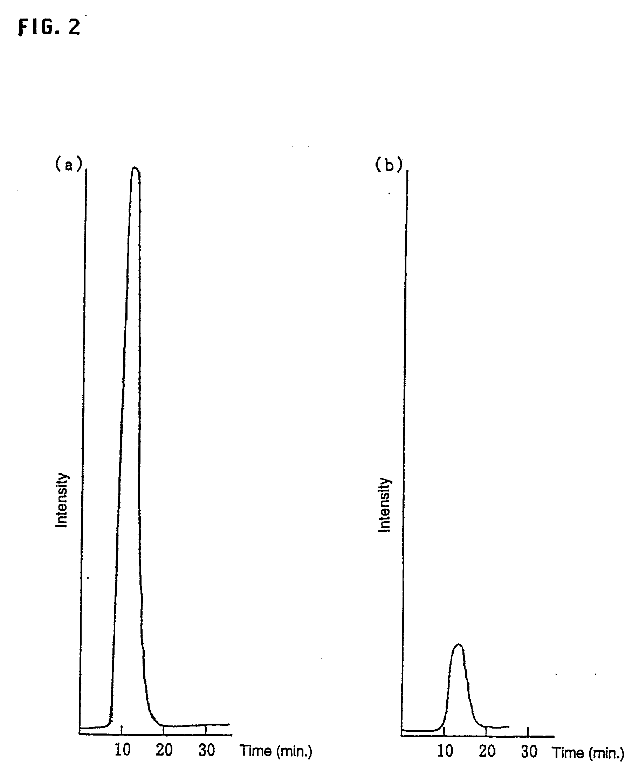

On the other hand, active TGF-β₁ was dissolved in 5 mM HCl to prepare a solution of active TGF-β₁ having a concentration of 0.3 g/liter of active TGF-β₁. The solution is a standard sample. Using the standard sample, each eluate as collected in the above was analyzed through linear gradient HPLC using from 0 to 350 mM KH₃PO₄ buffer (pH 7.4) for from 0 to 30 minutes. The HPLC column used herein had a size of 150 mm x 4 mm φ and was filled with 2 g of the above-mentioned, OH-carbonated hydroxyapatite. The results of this analysis verified that the peaks for the eluates (see Fig. 2(a) and Fig. 2(b)) resulted from the active TGF-β1 in the samples. The results of the elution of the samples from the cancer patient and those from the healthy person are shown in Fig. 2(a) and Fig. 2(b), respectively.

Comparing the peak in Fig. 2(a) and that in Fig. 2(b), it is aparent that the former is about 5 times higher than the latter. Thus, it is confirmed that the peak as detected from the serum samples of the cancer patient is higher than that as detected from the healthy person. From this, it is understood that the method as tested herein can produce an extremely distinct result in the detection of cancer.

## Claims

1. Cartridge column (1) for detecting active TGF-β1, comprising an inlet (2) and an outlet (3) for a liquid sample,
**characterized in that**
it has an ultrafilter (4) disposed at the inlet (2) and an adsorbent dispersion-preventing filter (5) disposed at the outlet (3), and that an adsorbent (6) for active TGF-β1 comprising, as active ingredient, an OH-carbonated hydroxyapatite with OH groups being partly or wholly substituted with CO₃ groups is present between the ultrafilter (4) and the filter (5).

2. Cartridge column as claimed in claim 1, characterized in that it is provided at the inlet for a liquid sample with means (7) for connecting it to the tip of a syringe.

3. Method for detecting cancer using a cartridge column of claim 1 or 2, in which a body fluid sample is introduced into the cartridge column (1) through the inlet (2) and diffused therein to make the sample brougth into contact with adsorbent (6) for active TGF-β1 that exists in the cartridge column, whereupon the active TGF-β1 existing in the sample is selectively adsorbed by the adsorbent, whereafter the thus-adsorbed active TGF-β1 is eluted with a buffer, and the active TGF-β1 in the resulting eluate is detected.
